# EUROPEAN PATENT APPLICATION

(11) **EP 4 219 738 A1**
(43) Date of publication of application: **02.08.2023**
(21) Application number: 20954643.1
(22) Date of filing: 25.09.2020
(51) Int. Cl.: C12Q 1/68

(54) **DETECTION METHOD FOR TUMOR-SPECIFIC T CELLS**

(71) Applicant: Suzhou Ersheng Biopharmaceutical Co., Ltd, Suzhou, Jiangsu 215125 (CN)
(72) Inventor: LIU, Mi, Suzhou, Jiangsu 215125 (CN)
(74) Representative: Betten & Resch
(86) International application number: PCT/CN2020/117952
(87) International publication number: WO 2022/061791

(57) **Abstract**

Disclosed is a detection method for tumor-specific T cells. The detection method comprises: collecting whole-cell components of tumor cells or tumor tissues, using free whole-cell components or loading whole-cell lysate components on nano/micron particles, then performing co-incubating with peripheral immune cells, and after the cancer-specific T cells are activated, detecting specific molecules of the tumor-specific T cells, so that the content of the cancer-specific T cells in peripheral tissues such as peripheral blood can be determined. The whole-cell lysate components in the present invention are water-soluble components and non-water-soluble components, and are in a free state or are loaded on nano particles or micron particles. The loading mode is that: the whole-cell water-soluble components and non-water-soluble components are respectively or simultaneously encapsulated inside the particles, and/or respectively or simultaneously loaded on the surfaces of the particles. The detection method comprises flow cytometry, enzyme-linked immunospot assay, enzyme-linked immunosorbent assay, colloidal gold immunochromatography, gene detection technology, etc.

## Description

### FIELD OF THE INVENTION

The present invention belongs to the field of immunotherapy and immunodetection, and in particular relates to a method for detecting tumor-specific T cells based on whole cells.

### BACKGROUND OF THE INVENTION

In recent years, immune technology has developed very rapidly, especially in the field of cancer immunotherapy. With the increasing awareness of cancer, people have found that the human immune system and various immune cells play a key role in the process of inhibiting the occurrence and development of cancer. Recently, PD-1 antibody therapy, CAR-T and other therapies have been approved for clinical use, with good clinical effects. However, cancer immunotherapy with a cancer vaccine, a PD-1 antibody and the like is only effective for some patients. Therefore, how to judge the effectiveness of immunotherapy drugs and the prognosis of patients before or during drug use is very critical.

The technique herein discloses a detection particle that can be effectively used for detecting the content of tumor-specific T cells, a corresponding preparation method thereof, a kit including the detection particle, and a detection method using the detection particle for detecting the content of tumor-specific T cells. The detection particle is used to activate tumor-specific T cells for the detection of the content of tumor-specific T cells based on any one or more of the cell secretions secreted by activated tumor-specific T cells, the proliferation status of activated tumor-specific T cells, or the cell surface markers of activated tumor-specific T cells in the sample to be tested. Immunotherapy relies on T cells activated by cancer specific/associated antigens in the immune system to kill tumor cells, so the content of cancer-specific T cells in patients is closely related to the efficacy of immunotherapy. However, there is a lack of effective means to comprehensively and accurately detect the content of cancer-specific T cells in patients' peripheral blood.

### Summary of the invention

### TECHNICAL ISSUES

The present invention provides a method for detecting tumor-specific T cells and their contents in peripheral tissues, which can provide reference information for the prognosis of cancer patients. Tumor-specific T cells are activated after being co-incubated with tumor cells, tumor tissue whole cells, tumor cell lysate components, tumor tissue whole-cell lysate components, or nano/micron particles loaded with lysate components, and secrete or express some specific molecules. The content of tumor-specific T cells can be determined by detecting these specific molecules secreted or expressed. The key technology is the activation of T cells.

### Solution to the problem

### TECHNICAL SOLUTION

The present invention applies the following technical solution: A method for detecting tumor-specific T cells is provided, comprising the following steps: incubating activators with peripheral immune cells, and then detecting specific molecules of the tumor-specific T cells, thus achieving detection of the tumor-specific T cells.

A method for detecting the content of tumor-specific T cells is provided, comprising the following steps: incubating activators with peripheral immune cells, then detecting specific molecules of the tumor-specific T cells, and then obtaining the content of tumor-specific T cells according to the ratio of the number of tumor-specific T cells to the number of peripheral immune cells.

In the present invention, the activators include tumor cells, tumor tissue whole cells, tumor cell lysate components, and tumor tissue whole-cell lysate components, and may also include immunoadjuvants; among them, the lysate components in tumor cell lysate components and tumor tissue whole-cell lysate components can be either water-soluble or non-water-soluble components of lysates, preferably water-soluble and non-water-soluble lysate components.

In the present invention, the activators can be free cells or free lysate components, or lysate components loaded on nano/micron particles; the free lysate components or the lysate components loaded on nano/micron particles are preferred; and the lysates are loaded inside and/or on the surfaces of nano/micron particles. The ways in which the lysate components are loaded inside and/or on the surfaces of nano/micron particles include, but are not limited to, non-covalent bond adsorption, electrostatic interaction, hydrophobic interaction, hydrogen bond interaction, covalent bond, etc.. The present invention can simultaneously use nano/micron particles loaded with water-soluble components and nano/micron particles loaded with non-water-soluble components, or nano/micron particles together loaded with water-soluble and non-water-soluble components, or nano/micron particles loaded only with water-soluble components, or nano/micron particles loaded only with non-water-soluble components.

In the present invention, incubation is carried out under the conditions that cells can survive, such as 4°C-60°C, preferably 37°C; and the incubation time is 1-100 h, such as 5-70 h, preferably 10-50 h.

In the present invention, the nano/micron particles can be organic materials, inorganic materials or biological materials, such as synthetic polymer materials, natural polymer materials or inorganic materials. The nano/micron particles are nano particles or micron particles, wherein the particle size of nano particles is 1-1,000 nm, preferably 30-800 nm, and further preferably 50-600 nm; and the particle size of micron particles is 1-1,000 µm, preferably 1-100 µm, further preferably 1-10 µm, most preferably 1-5 µm. The specific preparation methods of nano/micron particles are of the prior art, including a solvent evaporation method, a dialysis method, an extrusion method, a hot melt method, etc.. There is no limit to the shape of nano/micron particles, which can be spherical, ellipsoidal, barrel-shaped, polygonal, linear, worm-shaped, square, triangular, butterfly-shaped, disk-shaped, etc..

The method of loading cell lysate components onto nano/micron particles in the present invention is a solvent evaporation method such as a double emulsion method, or other methods that can load cell lysates onto nano/micron particles. Specifically, when the activators are the cell lysate components loaded on nano/micron particles, the preparation method is as follows: adding an aqueous phase solution to an organic phase solution of a nano/micron particle material, then performing ultrasonic treatment or stirring or homogenization treatment, then adding the obtained sample to a first emulsifier solution, then performing ultrasonic treatment or stirring or homogenization treatment, then adding the obtained sample to a second emulsifier solution, and then stirring to obtain nano/micron particles loaded with the cell lysate components as activators. For example, the following steps are included: (1) adding an aqueous phase solution to an organic phase solution of a polymer material, performing ultrasonic treatment or stirring or homogenization treatment, then adding the obtained sample to a first emulsifier solution, then performing ultrasonic treatment or stirring or homogenization treatment, then adding the obtained samples to a second emulsifier solution and stirring, followed by centrifuging and then resuspending the precipitate to obtain a residue, or ultrafiltering to obtain a residue.

(2) freeze-drying the residue from step (1), and re-dispersing it in a dispersion solution; or dispersing the residue from step (1) in a dispersion solution, and adding an aqueous phase solution to mix and then stand to obtain nano/micron particles as activators.

As described above, after the addition to a second emulsifier solution, stirring and then centrifuging or ultrafiltering, nano/micron particles loaded inside with the lysate components or the lysate components/immunoadjuvants are obtained. Further, the lysate components or the lysate components/immunoadjuvants are loaded on the surfaces of nano/micron particles loaded inside with the lysate components or the lysate components/immunoadjuvants.

The aqueous phase solution is a lysate component solution, or a lysate component/immunoadjuvant solution; the ultrasonic treatment is carried out by the probe ultrasonic treatment or any other ultrasonic method; the stirring is mechanical stirring, magnetic stirring, etc.; and the homogenization treatment is high-pressure homogenization treatment or high-shear homogenization treatment.

Preferably, when the aqueous phase solution is a lysate component solution, the concentration of protein and peptides are greater than 1 ng/mL, preferably 1-100 mg/mL; when the aqueous phase solution is a lysate component/immunoadjuvant solution, the concentration of protein and peptides are greater than 1 ng/mL, preferably 1-100 mg/mL, and the concentration of immunoadjuvant is greater than 0.01 ng/mL, preferably 0.01-20 mg/mL. In the organic phase solution of a polymer material, the solvent is DMSO, acetonitrile, ethanol, chloroform, methanol, DMF, isopropanol, dichloromethane, propanol, ethyl acetate, etc., preferably dichloromethane; and the concentration of the polymer material is 0.5-5,000 mg/mL, preferably 100 mg/mL. The first emulsifier solution is preferably a polyvinyl alcohol aqueous solution with a concentration of 10-50 mg/mL, preferably 20 mg/mL. The second emulsifier solution is preferably a polyvinyl alcohol aqueous solution with a concentration of 1-20 mg/mL, preferably 5 mg/mL. The dispersion solution is a PBS buffer solution or normal saline or pure water.

Preferably, when the stirring is mechanical or magnetic stirring, the stirring speed is greater than 50 rpm, e.g. 50-1500 rpm, and the stirring time is greater than 1 min, e.g. 0.5-5 h; during the ultrasonic treatment, the ultrasonic power is 50-500 W, and the ultrasonic time is greater than 0.1 s, e.g. 2-200 s; during the homogenization treatment, a high-pressure/ultrahigh-pressure homogenizer or a high-shear homogenizer shall be used, with the pressure greater than 20 psi for the high-pressure/ultrahigh-pressure homogenizer and the rotational speed greater than 1,000 rpm for the high-shear homogenizer. The ultrasonic treatment or stirring or homogenization treatment is performed for achieving a nanometer of particles, and the size of the prepared nanoparticles can be controlled by controlling the ultrasonic time or the stirring speed or the pressure and time of homogenization treatment, too large or too small of which will make the particle size change.

In the present invention, the volume ratio of the aqueous phase solution to the organic phase solution of a polymer material is 1 : (1.1-5,000), preferably 1 : (1.5-500); the volume ratio of the organic phase solution of a polymer material to the first emulsifier solution is 1 : (1.1-1,000), preferably 1 : (1.5-500); the volume ratio of the first emulsifier solution to the second emulsifier solution is 1 : (1.5-2,000), preferably 1 : (2-500); and the volume ratio of the dispersion solution to the aqueous phase solution is (1:10,000)-(10,000:1), preferably (1:100)-(100:1), most preferably (1:30)-(30:1).

In the present invention, cancer cells or tumor tissue whole cells are in a free state. The tumors include blood tumors and solid tumors, such as endocrine system tumors, nervous system tumors, reproductive system tumors, digestive system tumors, respiratory system tumors, blood cancer, skin cancer, breast cancer, lung cancer, liver cancer, stomach cancer, pancreatic cancer, brain cancer, colon cancer, prostate cancer, rectal cancer, head and neck cancer, kidney cancer, bone cancer, nasal cancer, bladder cancer, thyroid cancer, esophageal cancer, cervical cancer, ovarian cancer, uterine cancer, pelvic cancer, testicular cancer, penis cancer, lymphatic cancer, tongue cancer, gingival cancer, retinoblastoma, and sarcoma.

A solubilizing solution of the present invention for dissolving the non-water-soluble lysate components is a urea aqueous solution, a guanidine hydrochloride aqueous solution, a sodium deoxycholate aqueous solution, an SDS aqueous solution, a glycerin aqueous solution, an alkaline solution, an acidic solution, a protein degrading enzyme aqueous solution, an albumin aqueous solution, a lecithin aqueous solution, an inorganic salt solution, a polyethylene glycol octyl phenyl ether (Triton) aqueous solution, dimethyl sulfoxide (DMSO), acetonitrile, ethanol, methanol, N,N-dimethyl formamide (DMF), propanol, isopropanol, Tween, acetic acid, cholesterol, amino acid, glycoside, and choline; and the non-water-soluble lysate components can be dissolved in the solubilizing solution, or an organic solvent such as DMSO, glycerin, acetonitrile, ethanol, methanol, DMF, isopropanol, dichloromethane, propanol, ethyl acetate, etc.

In the present invention, after being activated by the activators, T cells secrete specific molecules including proteins, peptides, nucleic acids, sugars, or lipids; the specific molecules can be located in the cell membrane, cytoplasm, organelle or nucleus after being expressed; and tumor-specific T cells can be identified or quantified by detection, the detection methods comprising, but not limited to, flow cytometry, enzyme-linked immunospot assay, enzyme-linked immunosorbent assay, colloidal gold immunochromatography, gene detection technology, and multicytokine detection technology.

The immunoadjuvant of the present invention is an immunopotentiator or an immunosuppressant; and the immunopotentiator or immunosuppressant can be loaded only inside the nano/micron particles, only on the surfaces of nano/micron particles, or together inside and on the surfaces of nano/micron particles. The immunopotentiator is used to enhance the detection of immune cells that can secrete or express IFN-γ, IL-12 and other pro-inflammatory cell markers; and the immunosuppressant is used to enhance the detection of immune cells that can secrete or express IL-10 and other anti-inflammatory cell markers.

### BENEFICIAL EFFECTS

The present invention provides a method for activating the cancer-specific T cells in peripheral tissues by using free whole-cell components or whole-cell lysate components loaded on particles, detects the content of activated cancer-specific T cells by conventional detection technology, and can provide information support for the efficacy of cancer immunotherapy. When polypeptide antigen is used to stimulate and activate cancer-specific T cells in the prior art, the inaccuracy of cancer-specific T cells activated and subsequently detected will affect the scheme design and treatment effect of the subsequent immunotherapy. The present invention loads the free whole-cell components of cancer cells or tissues or the whole-cell components into nano/micron particles to activate cancer-specific T cells and detect the content of activated cancer-specific T cells, making the detected content of cancer-specific T cells more extensive and accurate.

### BRIEF DESCRIPTION OF THE DRAWINGS

In order to make the examples of the present invention or the technical solutions in the prior art more clearly explained, the drawings needed in the examples or the prior art will be briefly introduced in the following description.
Fig. 1 is a schematic diagram of the preparation process of the activator of the present invention.
Fig. 2 is a structural diagram of nano/micron particles loaded with water-soluble and non-water-soluble cell components.
Fig. 3 is a structural diagram of nano/micron particles loaded with water-soluble and non-water-soluble cell components.
Fig. 4 is a structural diagram of nano/micron particles loaded with water-soluble and non-water-soluble cell components.
Fig. 5 is a structural diagram of nano/micron particles loaded with water-soluble and non-water-soluble cell components.
Fig. 6 shows the experimental results of melanoma in Example 1.
Fig. 7 shows the experimental results of breast cancer in Example 2.
Fig. 8 shows the experimental results of melanoma in Example 3.
Fig. 9 shows the experimental results of lung cancer in Example 4.

In the following experimental results, each data point in the tumor growth inhibition experimental graph is a mean ± standard error of mean (mean ± SEM), and other experimental data points are a mean ± standard deviation (mean ± SD); the significant difference in the tumor growth inhibition experiment is analyzed by an ANOVA method, and the significant difference in other experiments is analyzed by a t-test; * means that there is a significant difference (P < 0.05) between this group and the control group, ** means that there is a significant difference (P < 0.01) between this group and the control group, and *** means that there is a significant difference (P *<* 0.0001) between this group and the control group.

### DETAILED DESCRIPTION OF THE EMBODIMENTS

The present invention discloses a method for detecting the content of tumor-specific T cells in peripheral tissues to predict the prognosis of patients, which is helpful for the diagnosis and treatment of diseases. Those skilled in the art can learn from this article and appropriately improve the process parameters. In particular, it should be noted that all similar replacements and modifications are obvious to those skilled in the art and are considered to be included in the present invention. The methods and products of the present invention have been described through preferred examples. It is obvious that those skilled in the art can change or appropriately modify and combine the methods described herein without departing from the content, spirit and scope of the present invention, so as to realize and apply the technology of the present invention.

The present invention discloses a technology for detecting tumor-specific T cells and their contents in peripheral tissues, which comprises the following steps: incubating activators with peripheral immune cells, and then detecting specific molecules of the tumor-specific T cells, thus achieving detection of the tumor-specific T cells; and incubating activators with peripheral immune cells, then detecting specific molecules of the tumor-specific T cells, and then obtaining the content of tumor-specific T cells according to the ratio of the number of tumor-specific T cells to the number of peripheral immune cells.

The present invention uses tumor tissue whole cells or tumor cells for detection, which can be divided into three steps: (1) collecting tumor cells or tumor tissues; (2) co-incubating the tumor cells or tumor tissue whole cells with samples of peripheral tissues containing immune cells such as T cells for more than 10 min, such as 16 h; and (3) using flow cytometry, enzyme-linked immunospot assay (ELISPOT), enzyme-linked immunosorbent assay (ELISA), multicytokine assay and the like to detect the specific molecules that mark the activation of T cells. The specific molecules can be secreted outside T cells, and can be expressed inside or on the surfaces of T cells. The specific molecules are proteins, nucleic acids, sugars, or lipids.

The present invention uses nano/micron particles loaded with cancer cell lysate components or tumor tissue lysate components for detection, which can be divided into four steps: (1) collecting tumor tissue whole cells or tumor cells; (2) preparing tumor cell lysate components or tumor tissue whole-cell lysate components, which can be further loaded on nano/micron particles; (3) co-incubating the nano/micron particles loaded with lysate components or lysate components with samples containing T cells and other immune cells in peripheral tissues for more than 10 min, such as 16 h; and (4) using flow cytometry, ELISPOT, ELISA, multicytokine assay and the like to detect the specific molecules that mark the activation of T cells. The specific molecules can be secreted outside T cells, and can be expressed inside or on the surfaces of T cells. The specific molecules are proteins, nucleic acids, sugars, or lipids.

In the present invention, tumor cells or tumor tissue whole cells can be used after inactivation or (and) denaturation treatment before or (and) after cell lysis, or can be used directly without any inactivation or (and) denaturation treatment before or (and) after cell lysis. The inactivation or (and) denaturation treatment methods can be ultraviolet irradiation and high-temperature heating. In the actual application process, the inactivation or denaturation treatment methods such as radiation irradiation, high pressure, freeze-drying and formaldehyde can also be used. Those skilled in the art can understand that they can make appropriate adjustments in the actual application process according to the specific situation.

Further, the activator includes an immunoadjuvant, which is an immunosuppressant or an immunopotentiator; and the activator can be loaded only inside the nano/micron particles, only on the surfaces of nano/micron particles, or simultaneously inside and on the surfaces of nano/micron particles.

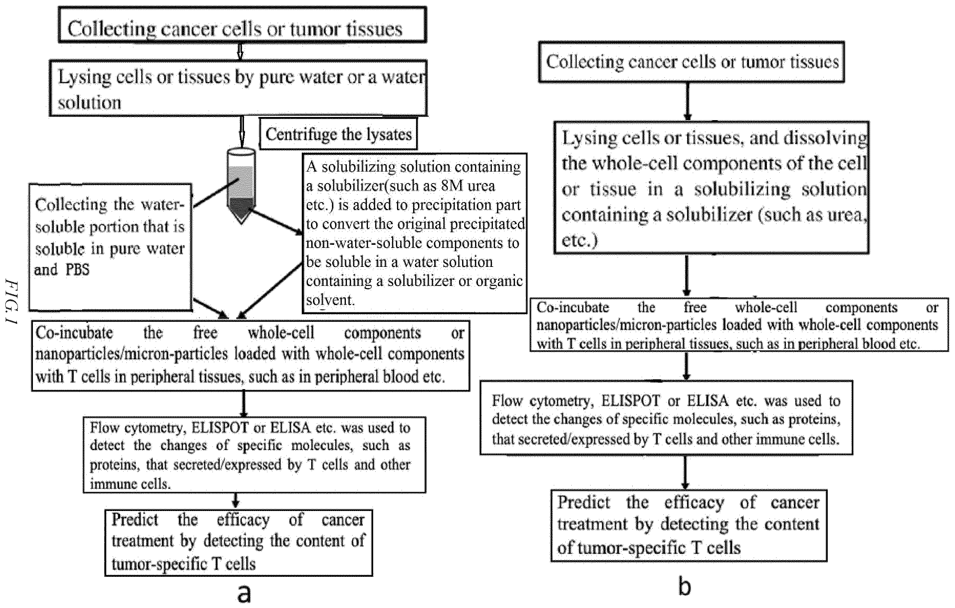

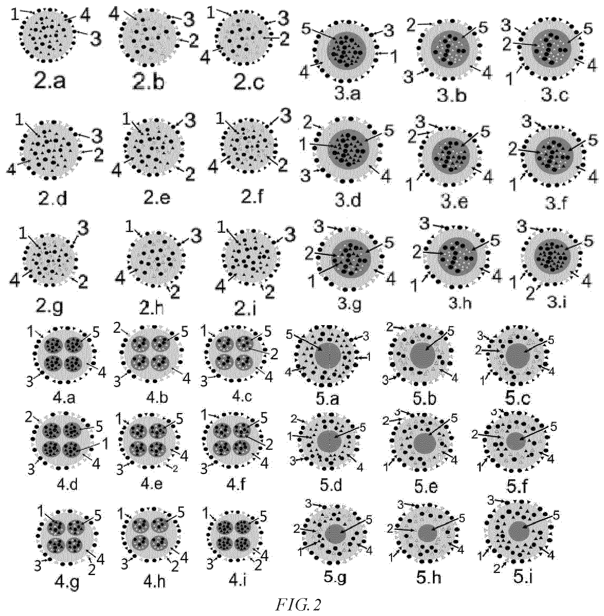

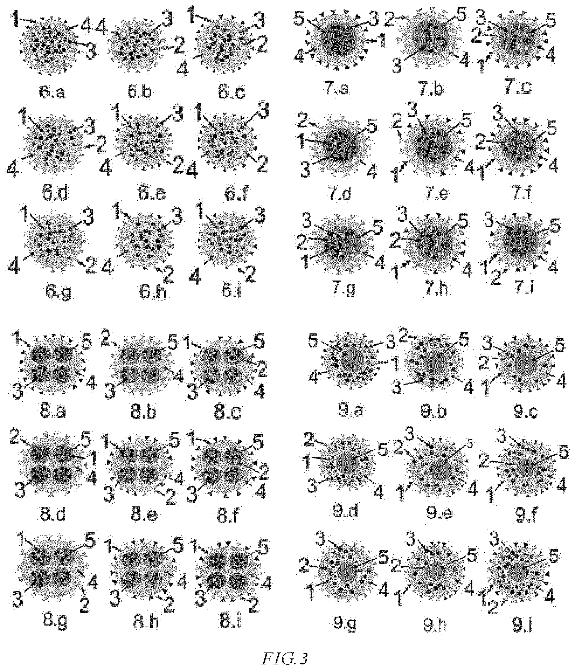

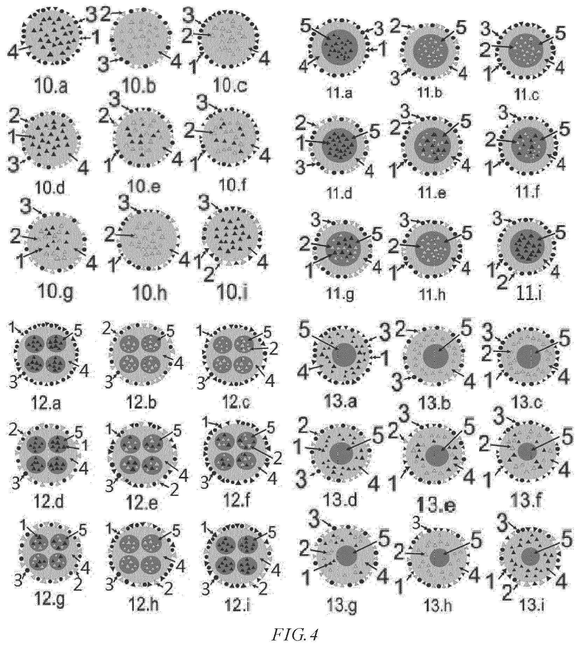

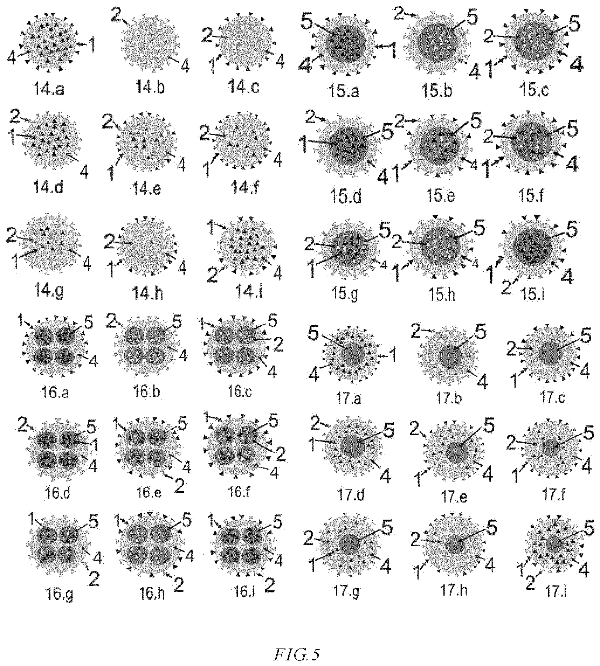

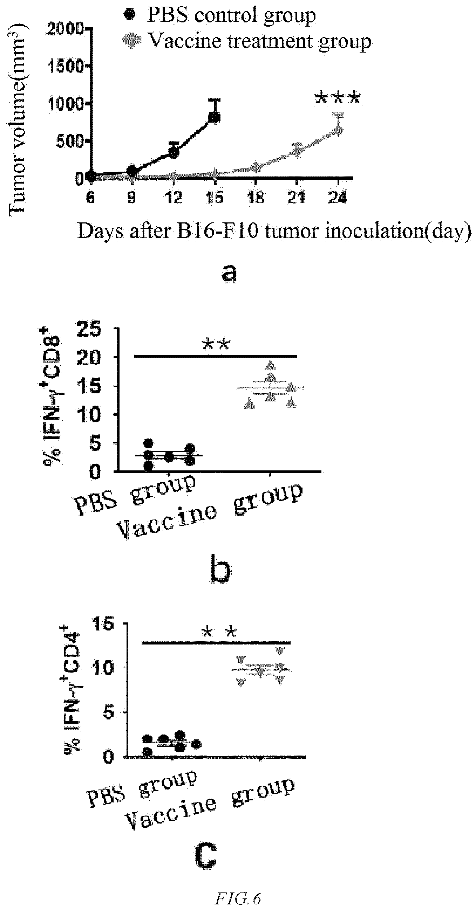

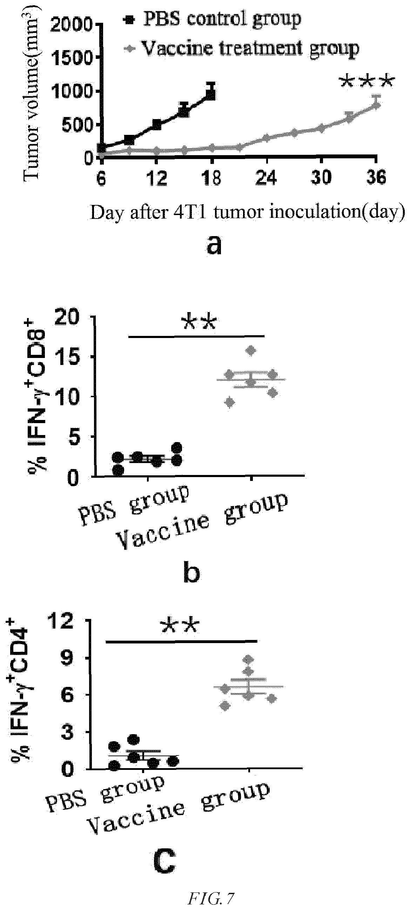

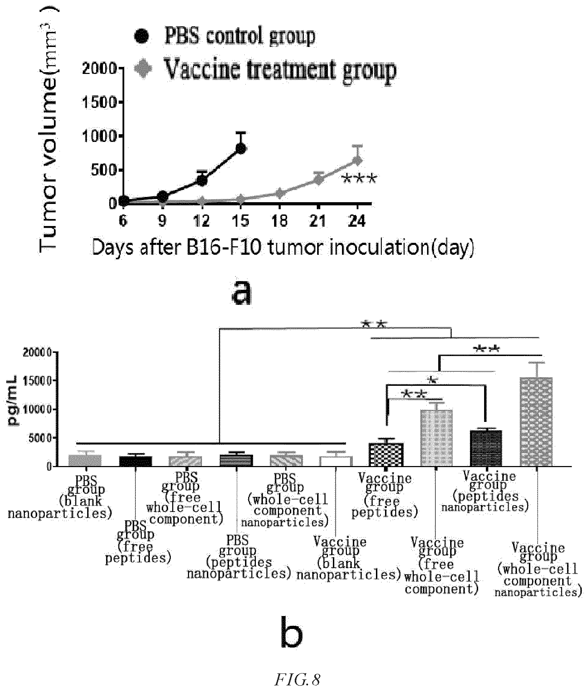

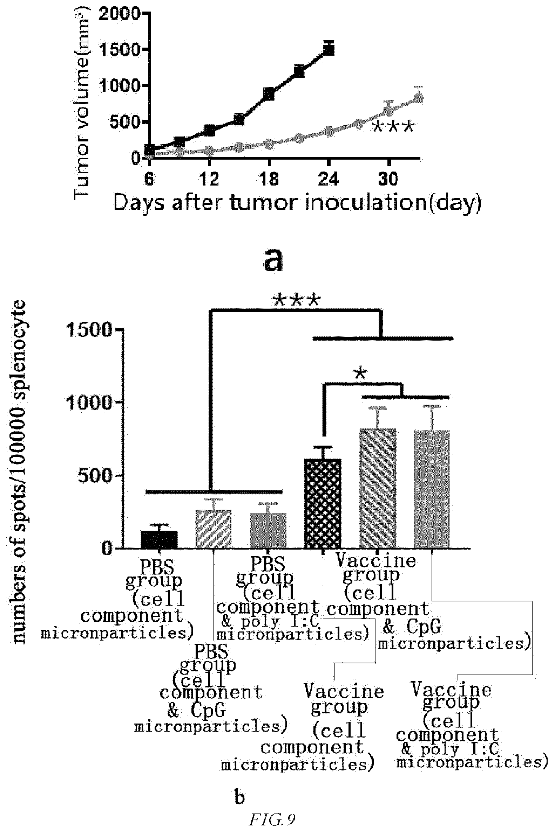

Fig. 1 is a schematic diagram of the preparation process of the activator of the present invention; and Figs. 2-5 are a structural diagram of nano/micron particles loaded with whole cells. In the actual application process, only the nano/micron particles with a specific structure can be used, or the nano/micron particles with two or more different structures can be used at the same time. In Fig. 2, the immunoadjuvant is contained inside and on the surfaces of nano/micron particles; in Fig. 3, the immunoadjuvant is only distributed inside the nano/micron particles; in Fig. 4, the immunoadjuvant is contained only on the surfaces of nano/micron particles; in Fig. 5, the immunoadjuvant is contained neither inside nor on the surfaces of nano/micron particles; in 2a-2j of Fig. 2, 6a-6j of Fig. 3, 10a-10j of Fig. 4, and 14a-14j of Fig. 5, when the water-soluble or non-water-soluble components in the cells or tissue components loaded on the nano/micron particles are distributed inside the nano/micron particles, no obvious core is formed; in 3a-3j of Fig. 2, 7a-7j of Fig. 3, 11a-11j of Fig. 4, and 15a-15j of Fig. 5, when the water-soluble or non-water-soluble components in the cells or tissue components loaded on the nano/micron particles are distributed inside the nano/micron particles, a core is formed during the preparation process or by using polymers or inorganic salts; in 4a-4j of Fig. 2, 8a-8j of Fig. 3, 12a-12j of Fig. 4, and 16a-16j of Fig. 5, when the water-soluble or non-water-soluble components in the cells or tissue components loaded on the nano/micron particles are distributed inside the nano/micron particles, a plurality of cores are formed during the preparation process or by using polymers or inorganic salts; and in 5a-5j of Fig. 2, 9a-9j of Fig. 3, 13a-13j of Fig. 4, and 17a-17j of Fig. 5, when the water-soluble or non-water-soluble components in the cells or tissue components loaded on the nano/micron particles are distributed inside the nano/micron particles, they are located in the outer layer of the formed core. In each figure, 1 represents the water-soluble components in the cells or tissue components; 2 represents the non-water-soluble components in the cells or tissue components; 3 represents the immunoadjuvant; 4 represents the nano/micron particles; 5 represents the core of nano/micron particles; a represents that what are loaded inside and on the surfaces of nano/micron particles are water-soluble components in the cells or tissue components; b represents that what are loaded inside and on the surfaces of nano/micron particles are non-water-soluble components in the cells or tissue components; c represents that what are loaded inside the nano/micron particles are non-water-soluble components in the cells or tissue components, and what are loaded on the surfaces of nano/micron particles are water-soluble components in the cells or tissue components; d represents that what are loaded inside the nano/micron particles are water-soluble components in the cells or tissue components, and what are loaded on the surfaces of nano/micron particles are non-water-soluble components in the cells or tissue components; e represents that the water-soluble and non-water-soluble components in the cells or tissue components are simultaneously loaded inside the nano/micron particles, and are simultaneously loaded on the surfaces of nano/micron particles; f represents that the water-soluble and non-water-soluble components in the cells or tissue components are simultaneously loaded inside the nano/micron particles, while only the water-soluble components in the cells or tissue components are loaded on the surfaces of nano/micron particles; g represents that the water-soluble and non-water-soluble components in the cells or tissue components are simultaneously loaded inside the nano/micron particles, while only the non-water-soluble components in the cells or tissue components are loaded on the surfaces of nano/micron particles; h represents that only the non-water-soluble components in the cells or tissue components are loaded inside the nano/micron particles, while the water-soluble and non-water-soluble components in the cells or tissue components are simultaneously loaded on the surfaces of nano/micron particles; and i represents that only the water-soluble components in the cells or tissue components are loaded inside the nano/micron particles, while the water-soluble and non-water-soluble components in the cells or tissue components are simultaneously loaded on the surfaces of nano/micron particles.

In some examples, first the cell lysate components can be loaded into the nano/micron particles, with the immunoadjuvant simultaneously loaded; and then the cell lysate components are loaded onto the surfaces of nano/micron particles, with the immunoadjuvant simultaneously loaded onto the surfaces of nano/micron particles. In practical applications, it is possible to directly lyse tumor cells or tumor tissue whole cells with a solubilizing solution (such as an 8 M urea aqueous solution or a 6 M guanidine hydrochloride aqueous solution), then directly dissolve the cell lysate components, and then load them onto the nano/micron particles.

The method of loading cell lysate components onto nano/micron particles is a solvent evaporation method, or any other methods that can load the cell lysate components onto the nano/micron particles. In some embodiments, the compound emulsion method in the solvent evaporation method is used to prepare the nanoparticles; the material used to prepare the nano/micron particles is a polymer material, such as the organic macromolecule poly(lactic-co-glycolic acid) (PLGA) with a molecular weight of 24-38 Kda; the PLGA material is biodegradable and has been approved by FDA as a drug dressing, suitable for preparing the nano/micron particles; and the immunoadjuvant used is poly(I:C) or CpG.

Preferably, "freeze-drying the residue from step (1)" is to resuspend the residue from step (1) in a freeze-drying protective agent aqueous solution and then freeze-dry it; and the freeze-drying protective agent is preferably trehalose or sucrose, with a concentration of 2-8 wt%, preferably 3-6 wt%.

In the present invention, the compound emulsion method is used for the preparation of nanoparticles, with any other commonly used nano/micron particle preparation method also allowed to be used in practical applications; PLGA is used as the material for the preparation of nano/micron particles, with any other material that can be used to prepare the nano/micron particles also allowed to be used in practical applications; nano particles are used in some examples, and micron particles are used in some other examples, with those skilled in the art allowed to use nano/micron particles in practical applications according to the actual situation; flow cytometry is used as the detection method in some examples, and ELISPOT or ELISA is used as the detection method in some other examples, with multi cytokine assay and other detection methods also allowed to be used in practical applications according to the actual situation; interferon-γ (IFN-γ) is used as the specific molecule of tumor-specific T cells in some examples, with any other specific molecules, either being secretory or being membrane binding, including proteins, nucleic acids, sugars and lipids, also allowed to be used in practical applications; and the specific cytokine detected in this example is pro-inflammatory, with anti-inflammatory cytokines, such as IL-10 and TGF-β, also allowed to be used in practical applications.

In the present invention, poly(I:C) and CpG are used as an immunoadjuvant, with no immunoadjuvant or any other immunoadjuvant with an immunopotentiating/immunosuppressing function such as the following also allowed to be used in practical applications: a pattern recognition receptor agonist, a BCG cell-wall skeleton, BCG methanol extraction residue, BCG cell-wall acyl dipeptide, mycobacterium phlei, polyantigen A, mineral oil, a virus-like particle, an immunopotentiating reconstituted influenza virosome, cholera enterotoxin, a saponin and its derivatives, BCG, Resiquimod, thymosin, newborn bovine liver active peptide, imiquimod, polysaccharide, curcumin, an immunoadjuvant poly ICLC, corynebacterium parvum vaccine, a hemolytic streptococcus preparation, a coenzyme Q10, levamisole, polycytidylic acid, interleukin, interferon, polyinosinic acid, polyadenylate, alum, aluminum phosphate, lanolin, vegetable oil, endotoxin, a liposome adjuvant, GM-CSF, MF59, a double stranded RNA, a double stranded DNA, aluminum hydroxide, CAF01, ginseng, and astragalus and other effective ingredients of traditional Chinese medicine. As for immunoadjuvants, they can be added or not added in the present invention. When added, the immunoadjuvants are at least one of immunoadjuvants from microorganisms, products of human or animal immune system, intrinsic immune agonists, adaptive immune agonists, chemical synthetic drugs, fungal polysaccharides, and traditional Chinese medicines.

In order to further understand the present invention, the technical solutions in the examples of the present invention will be described clearly and completely in combination with the examples of the present invention. Obviously, the described examples are only part, not all, of the examples of the present invention. Based on the examples, all the other examples obtained by those skilled in the art without making creative efforts shall fall within the scope of protection of the present invention.

Unless otherwise specified, the specific methods used in the examples of the present invention are conventional methods, and the materials and reagents used can be obtained commercially. The nano/micron particle structures, preparation methods, methods of co-incubation with T cells in peripheral tissues, strategies for detecting activated T cells, etc. involved in the examples of the present invention are only representative ones; and the methods described in the present invention can also be used for other nano/micron particle structures, preparation methods, methods of co-incubation with T cells in peripheral tissues, and strategies for detecting activated T cells. The examples only list the application of the present invention in some cancers, but the present invention can also be used in other cancers. For the specific methods or materials used in the examples, those skilled in the art can, on the basis of the technical idea of the present invention, make conventional replacement choices according to the existing technologies, not limited to the specific records of the examples of the present invention.

### Example 1: Detection of tumor-specific T cells in peripheral tissues of melanoma-bearing mice

In this example, the mouse melanoma was used as a cancer model to illustrate the use of free cancer cell whole-cell lysates to detect the tumor-specific T cells in peripheral tissues and the content of tumor-specific T cells. Since the amount of peripheral blood of mice is not much and the number of peripheral immune cells in the peripheral blood is limited, while the splenocytes is rich and contains enough peripheral immune cells, so the peripheral immune cells in the spleen of mice were used for relevant detection in this example. The immune cells in the spleen belong to peripheral immune cells, and the immune cells in the peripheral blood also belong to peripheral immune cells. In clinical practice, the peripheral immune cells in human peripheral blood can be used for detection.

In this example, B16-F10 mouse melanoma cells were used as a tumor cell model. First, the B16-F10 cells were lysed to prepare the lysate components of B16-F10 cells; then, the free tumor cell lysate components were co-incubated with the peripheral immune cells overnight; and finally, flow cytometry was used to analyze the specific molecules (interferon γ) of tumor-specific T cells. The steps were specifically as follows: (1) Lysis of tumor cells and collection of components: collecting the B16-F10 cells, removing the culture medium and freezing the cells at -80°C, then adding ultrapure water and freezing and thawing the cells repeatedly for three times, and meanwhile performing 150 W ultrasonic treatment to destroy the lysed cells; after the cell lysis, centrifuging the lysates at a rotational speed of 12,000 rpm for 5 min, and then taking the supernatant as the water-soluble lysate components in the B16-F10 cells; and the precipitate was added with an 8 M urea aqueous solution to solubilize the precipitate, thus obtaining the non-water-soluble lysate components in the B16-F10 cells.

(2) Co-incubation of free tumor cell lysate components with peripheral immune cells: female C57BL/6 mice aged 6-8 weeks were selected to prepare melanoma-bearing mice. On Day 0, inoculating 150,000 B16-F10 cells subcutaneously into the lower right back of each mouse; on Days 4, 7, 10, 15 and 20, subcutaneously injecting the mice with PBS or an cancer nanovaccine loaded with whole-cell components of corresponding melanoma cancer cells. In the experiment, the tumor volume of mice was recorded every three days from day 6. The tumor volume was calculated by the formula v = 0.52 × a × b², where v being the tumor volume, a being the tumor length, and b being the tumor width. On day 18 or day 24, C57BL/6 mice in the PBS group or in the nanovaccine treatment group were sacrificed, and peripheral immune cells in their spleens were collected for subsequent parallel experiments.

The cells were resuspended in a DMEM culture medium containing 10% FBS with a concentration of 4 × 10⁶ cells/mL. And then 10% (by volume, based on the culture medium) of water-soluble lysate components (40 mg/mL) and 1% (by volume, based on the culture medium) of non-water-soluble lysate components (30 mg/mL) were added into the cells, followed by incubating at 37°C with 5% CO₂ for 20 h. The mouse splenocytes were collected by centrifuging at 400 g centrifugation after incubations.

(3) Detection of activated tumor-specific T cells by flow cytometry: first, the collected mouse spleen cells were treated with Fc block to avoid non-specific loading. And then, the anti-CD3 antibody, anti-CD4 antibody and anti-CD8 antibody were applied to carry out extracellular staining on the mouse splenocytes. After that, the cells were fixed and membranes of the cells were broken, followed by using the IFN-γ antibody to stain mouse splenocytes intracellularly. Finally, FACS AriaTMIII system was utilized to detect the mouse spleen cells, the FlowJo 10 software was applied to analyze the results. The ratio of the CD4⁺ T cells that could secrete IFN-γ after being activated to all the CD4⁺ T cells and the ratio of the CD8⁺ T cells that could secrete IFN-γ after being activated to all the CD8⁺ T cells were analyzed respectively.

(4) Experimental results: The above experimental results were shown in Fig. 6. In Fig. 6, a showed the inhibition effect of cancer vaccine treatment on the tumor growth rate (n ≥ 8), b showed the ratio of the activated tumor-specific CD8⁺ T cells in the peripheral immune cells of peripheral spleen after the incubation with the tumor tissue lysates to the CD8⁺ cells in spleen analyzed by flow cytometry, and c showed the ratio of the activated tumor-specific CD4⁺ T cells in the peripheral immune cells of peripheral spleen after the incubation with the tumor tissue lysates to the CD4⁺ cells in the peripheral immune cells of spleen analyzed by flow cytometry. As shown in Fig. 6, compared with the PBS blank control group, there were significantly more activated T cells after the peripheral immune cells of mice in the vaccine treatment group were co-incubated with the tumor cell lysates, which indicated that the content of tumor-specific T cells in the peripheral tissues of mice treated with the cancer vaccine increased significantly. It could be seen that the free whole cell of the present invention could be used to detect the content of tumor-specific T cells in the peripheral blood of cancer patients.

### Example 2: Detection of cancer-specific T cells in peripheral tissues of breast cancer-bearing mice

In this example, the mouse breast cancer was used as a cancer model to illustrate the use of free tumor tissue whole-cell lysates to detect the tumor-specific T cells in peripheral tissues and the content of tumor-specific T cells. The peripheral blood of mice is not much and the number of peripheral immune cells in the peripheral blood is limited, while the spleen is rich of blood flow and contains enough peripheral immune cells, so the peripheral immune cells in the spleen of mice were used for relevant detection in this example. In clinical practice, the peripheral immune cells in human peripheral blood can be used for detection.

In this example, 4T1 mouse breast tumor cells were used as a tumor cell model. First, the tumor tissue whole cells were lysed to prepare water-soluble and non-water-soluble components; then, the free tumor whole-cell lysate components were co-incubated with the peripheral immune cells overnight; and finally, flow cytometry was used to analyze the specific molecules (interferon γ) of tumor-specific T cells.
(1) Lysis of tumor tissues and collection of components: inoculating 400,000 4T1 breast tumor cells subcutaneously into the back of each BALB/c mouse, and sacrificing the mice when the tumors inoculated in each mouse grew to a volume of 200-1500 mm³, followed by collecting the tumor tissues. The tumor tissues were cut into pieces and then grinded, followed by going through a cell filter screen, adding pure water, and then repeatedly freezing and thawing for 5 times. After the cells in the tumor tissues were lysed, the cell lysates were centrifuged at a rotational speed of 3,000 g for 20 min, and then the supernatant was taken as the water-soluble components in the tumor tissue whole cells; an 8 M urea aqueous solution were added to the obtained precipitate to solubilize the precipitate, thus obtaining the non-water-soluble components.
(2) Co-incubation of free whole cell components with peripheral immune cells: selecting female BALB/c mice aged 6-8 weeks to prepare breast tumor-bearing mice. On day 0, inoculating 400,000 B16-F10 cells subcutaneously into the lower right back of each mouse; on days 4, 7, 10, 15 and 20, subcutaneously injecting the mice with PBS or an cancer nanovaccine loaded with whole-cell components of breast cancer cell. In the experiment, the tumor volumes of mice were recorded every three days from day 6. The tumor volume was calculated by the formula v = 0.52 × a × b², where v being the tumor volume, a being the tumor length, and b being the tumor width. On day 24, the mice were sacrificed in the vaccine treated group and the PBS group, and the immune cells in their spleens were collected.
   The cells were resuspended in an RPMI 1640 culture medium containing 10% FBS with a concentration of 4 × 10⁶ cells/mL. 5% (by volume, based on the culture medium) of water-soluble components (35 mg/mL) and 1% (by volume, based on the culture medium) of non-water-soluble components (35 mg/mL) dissolved in an 8 M urea solution were added into the cells, followed by incubating at 37°C with 5% CO₂ for 18 h. Then, the incubated mouse splenocytes were collected after 400 g centrifugation.
(3) Detection of activated cancer-specific T cells by flow cytometry: first, the mouse splenocytes were treated with Fc block to avoid non-specific loading. And then, the anti-CD3 antibody, anti-CD4 antibody and anti-CD8 antibody were applied to carry out extracellular staining on the mouse splenocytes. Then, the cells were and membranes of the cells were broken, followed by using the IFN-γ antibody for intracellular staining of the mouse splenocytes. Finally, FACS AriaTMIII system was utilized to detect the mouse splenocytes, and the FlowJo 10 software was applied to analyze the results. The ratio of the CD4⁺ T cells that could secrete IFN-γ after being activated to all the CD4⁺ T cells and the ratio of the CD8⁺ T cells that could secrete IFN-γ after being activated to all the CD8⁺ T cells were analyzed respectively.
(4) Experimental results: The above experimental results of breast cancer were shown in Fig. 7. In Fig. 7, a showed the inhibition effect of cancer vaccine treatment on the tumor growth rate (n ≥ 9), b showed the ratio of the activated cancer-specific CD8⁺ T cells in the peripheral immune cells of peripheral spleen after the incubation with the tumor tissue lysates to the CD8⁺ cells in the peripheral immune cells of spleen, and c showed the ratio of the activated cancer-specific CD4⁺ T cells in the peripheral immune cells of peripheral spleen after the incubation with the tumor tissue lysates to the CD4⁺ cells in the peripheral immune cells of spleen.

As shown in Fig. 7, compared with the PBS blank control group, there were significantly more activated T cells after the peripheral immune cells of mice, in the vaccine treatment group, were co-incubated with the tumor tissue whole-cell lysates. This indicated that the content of cancer-specific T cells in the peripheral tissues of mice treated with the cancer vaccine increased significantly. It could be seen that the free whole cell of the present invention could be used to detect the content of cancer-specific T cells in the peripheral blood of cancer patients.

### Example 3: Activation of tumor-specific T cells in peripheral tissues by nanoparticles loaded with melanoma tumor tissue lysate components

In this example, a mouse melanoma model was used to explain how to prepare nanoparticles loaded with melanoma tumor tissue whole-cell components and how to use the nanoparticles to activate the tumor-specific T cells in peripheral tissues. After T cells were activated, the content of activated tumor-specific T cells was detected by ELISA.

In this example, Enzyme-Linked ImmunoSorbent Assay (ELISA) was used to detect IFN-γ secreted by the activated T cells. In practical applications, other methods such as ELISPOT and flow cytometry can also be used to detect other substances secreted by activated T cells or expressed on the surfaces of cell membranes.

In this example, mouse B16-F10 melanoma cells were inoculated into C57BL/6 mice. Then the tumor tissues were extracted, and the water-soluble components in the tumor tissue lysate components and the original non-water-soluble components solubilized with 8 M urea solution were simultaneously loaded onto the inside and surfaces of the nanoparticles. PLGA was used as the framework material of nanoparticles to prepare nanoparticles loaded with water-soluble and non-water-soluble components of tumor tissue lysates by the solvent evaporation method, and such nanoparticles were utilized to activate the tumor-specific T cells in the peripheral tissues of mice.
(1) Lysis of tumor tissues and collection of different components: 150,000 B16-F10 melanoma cells were inoculated subcutaneously into the back of each C57BL/6 mouse, and the mice were sacrificed followed by extracting the tumor tissues when the tumors inoculated in each mouse grew to a volume of 200-1500 mm³. Tumor tissues were cut into pieces and grinded, followed by going through a cell filter screen. The sample was then added with pure water, and then repeatedly lyophilized for 5 times, with ultrasonic treatment performed at 150 W for 2 min at each thawing. After the cells in the tumor tissues were lysed, the cell lysates of tumor tissues were centrifuged at 100 g for 5 min, and then the supernatant was taken as the water-soluble components soluble in pure water in the tumor tissues; an 8 M urea aqueous solution was added to solubilized the the obtained precipitate, thus converting the original non-water-soluble components insoluble in pure water into components soluble in an 8 M urea aqueous solution. The water-soluble components of tumor tissue lysates obtained above and the original non-water-soluble components dissolved in an 8 M urea solution were utilized as the source of raw materials to prepare nanoparticles.
(2) Preparation of nanoparticles loaded with whole-cell components: In this example, the nanoparticles loaded with cell components and the blank control nanoparticles were prepared by the double emulsion method in the solvent evaporation method. The molecular weight of the material PLGA, used for preparing the nanoparticles, was 24-38 KDa, and the preparation method was as described previously. In addition, in this example, nanoparticles together loaded with four melanoma polypeptide antigens as follows were also prepared: Melan-A:26-35 (L27: GILTV), Melan-A: 51-73 (RR23: RNGYRALMDKSLHVGTQCALTRR), gp100:25-33 (EGSRNQDWL) and gp100: 44-59 (WNRQLYPEWTEAQRLD). The concentration of each peptide was 5 mg/mL in the preparation of nanoparticles. The steps were specifically as follows: 200 µL of the above water-soluble component solution (30 mg/mL) and 200 µL of the non-water-soluble component solution (30 mg/mL) were added to 1 mL of PLGA (100 mg) dichloromethane solution, and then conducting the conventional ultrasonic treatment for 30 s. And then the sample was mixed with 2.5 mL of polyvinyl alcohol aqueous solution (20 mg/mL), conducting the conventional ultrasonic treatment for 30 s. After that, the sample was mixed with 50 mL of polyvinyl alcohol aqueous solution (5 mg/mL), followed by stirring conventionally until the complete volatilization of organic solvent (dichloromethane). Subsequently, the sample was centrifuged at 12,000 rpm for 10 min. The supernatant was taking out and the precipitate was resuspended in 20 mL of trehalose aqueous solution (4 wt%). The sample was freeze-drying at -80°C, and then the sample was resuspended in 10 mL of normal saline. And then mixing with 0.5 mL of the above water-soluble component solution (30 mg/mL) and 0.5 mL of the non-water-soluble component solution (30 mg/mL), followed by standing for 60 s to obtain the activator of nanoparticles loaded with the lysate components inside and on the surfaces of the nanoparticles.
   The preparation of empty nanoparticles and nanoparticles loaded with the four polypeptide antigens was the same as above, with the lysate components replaced or not added.
   The average particle size of nanoparticles before being loaded with the lysate components on the surfaces thereof was about 280 nm; the particle size of nanoparticles after being loaded with the lysate components on the surfaces thereof was about 300 nm; and 150 µg of lysate components were loaded on per mg of PLGA nanoparticles. The particle size of blank nanoparticles was about 250 nm. The particle size of nanoparticles loaded with the polypeptide antigens was about 290 nm, and the total peptide loading capacity of 1 mg of PLGA nanoparticles was about 50 µg of polypeptide antigens.
(3) Nanoparticles activating tumor-specific T cells: female C57BL/6 mice aged 6-8 weeks were selected to prepare melanoma-bearing mice. On day 0, 150,000 B16-F10 melanoma cells were inoculated subcutaneously into the lower right back of each mouse. On days 4, 7, 10, 15 and 20, the mice were subcutaneously injected with PBS or cancer nanovaccines loaded with whole-cell components of cancer cells. In the experiment, the tumor volumes of mice were recorded every three days from Day 6 and the tumor volume was calculated by the formula v = 0.52 × a × b², where v being the tumor volume, a being the tumor length, and b being the tumor width. On day 18 or day 24, the mice in the PBS group or in the vaccine treatment group were sacrificed and the peripheral immune cells in their spleens were collected.
   The cells were resuspended in an RPMI 1640 culture medium containing 10% FBS with a concentration of 5 × 10⁶ cells/mL. Then, the nanoparticles loaded with the water-soluble and non-water-soluble components with a final concentration of 300 µg/mL were added into the sample; or the nanoparticles loaded with the polypeptide antigens with a final concentration of 300 µg/mL were added into the sample; or the blank nanoparticles of the same amount were added into the sample; or the free whole-cell lysate components of the same amount were added into the sample; or the free peptide antigen of the same amount were added into the sample. The sample were then incubated in an incubator at 37°C (5% CO₂) for 72 h and the samples were then centrifugated at 400 g for 5 min, followed by collecting the supernatant and analyzing the concentration of IFN-γ in the supernatant by the ELISA detection.
   In the ELISA detection method, the tumor-specific T cells, after being activated, would secrete specific cell secretions such as IFN-γ. The concentration of such specific cell secretions represented the content of activated tumor-specific T cells.
(4) Experimental results: The above experimental results of melanoma were shown in Fig. 8. In fig. 8, a showed the inhibition effect of cancer vaccine treatment on the tumor growth rate (n ≥ 8), and b showed the content of activated tumor-specific T cells in the peripheral immune cells of peripheral spleen after the incubation with free tumor tissue lysates or nanoparticles loaded with tumor tissue lysates analyzed by the ELISA detection.

As shown in Fig. 8, compared with the PBS blank control group and the blank nanoparticle group in the vaccine treatment group, the number of activated T cells was significantly higher in the vaccine treated group after the co-incubation of peripheral immune cells of mice with free whole-cell components of tumor tissue, or nanoparticles loaded with whole-cell components of tumor tissue, or free peptide antigens, or nanoparticles loaded with polypeptide antigens, which indicated that the content of tumor-specific T cells in the peripheral tissues of mice treated with the cancer vaccine increased significantly. It could be seen that the free whole-cell components of tumor tissue of the present invention could be used to detect the content of tumor-specific T cells in the peripheral blood of cancer patients. When free whole-cell components of tumor tissue or free polypeptide antigens were used to stimulate and activate T cells, the free whole-cell components could stimulate and activate more T cells; when nanoparticles loaded with whole-cell lysate components or nanoparticles loaded with polypeptide antigens were used to stimulate and activate T cells, the nanoparticles loaded with whole-cell lysate components could stimulate and activate more T cells; moreover, the nanoparticles loaded with whole-cell lysate components stimulated and activated more T cells than the free whole-cell lysate components.

### Example 4: Activation of tumor-specific T cells in peripheral tissues by micron particles loaded with lung cancer tumor tissue lysate components and immunoadjuvants

This example described, based mouse lung cancer, the preparation of micron particles loaded with lung cancer tumor tissue lysate components and immunoadjuvants, and the preparation of micron particles loaded only with lung cancer tumor tissue lysate components, so as to activate tumor-specific T cells in peripheral tissues; and ELISPOT was used to detect the content of tumor-specific T cells. This example tested the effects of adding with CpG or adding with poly I:C as the immunoadjuvant or without any immunoadjuvant, respectively.

This example used enzyme-linked immunospot assay (ELISPOT) to detect the specific molecule IFN-γ of activated tumor-specific T cells; in practical applications, flow cytometry, ELISA and other methods can also be used to detect other specific molecules of tumor-specific T cells.

In this example, mouse LLC lung tumor cells were inoculated into C57BL/6 mice and then the tumor tissues were extracted. The water-soluble components in tumor tissue lysate components and the non-water-soluble components dissolved in a 6 M guanidine hydrochloride solution were then obtained. PLGA was utilized as the framework material to prepare micron particles, loaded with water-soluble components of tumor tissue lysates and non-water-soluble components, by the solvent evaporation method. The water-soluble and non-water-soluble components in the tumor tissue lysates were simultaneously loaded inside and on the surfaces of micron particles; and in the micron particles containing an immunoadjuvant, the immunoadjuvant was only loaded inside the micron particles. These micron particles were then used to detect the tumor-specific T cells in the peripheral tissues of mice.
(1) Lysis of tumor tissues and collection of components: 2,000,000 LLC lung tumor cells were inoculated subcutaneously into the back of each C57BL/6 mouse, and the mice were sacrificed when the tumors inoculated in each mouse grew to a volume of 200-1500 mm³, followed by collecting the tumor tissues. The tumor tissues were cut into pieces and then grinded, followed by going through a cell filter screen and adding pure water. And then tumor tissue whole cells were inactivated and denatured by conventional ultraviolet irradiating and heating, followed by repeatedly lyophilizing for 5 times, with ultrasonic treatment performed at 250 W for 1 min at each thawing. After the tumor tissue whole cells were lysed, the cell lysates were centrifuged at a rotational speed of 5,000 rpm for 15 min. And then, the supernatant was taken as the water-soluble components in the tumor tissue whole cells; and a 6 M guanidine hydrochloride aqueous solution was added to the obtained precipitate to solubilize it, thus obtaining the non-water-soluble components.
(2) Preparation of micron particles loaded with whole-cell components: In this example, the micron particles loaded with cell lysates were prepared by the double emulsion method in the solvent evaporation method, and the molecular weight of the material PLGA used for preparing the micron particles was 24-38 KDa. The preparation method was same as described previously. The steps were specifically as follows: 150 µL of the above water-soluble component solution (60 mg/mL) or 200 µL of the non-water-soluble component solution (10 mg/mL) was added to 2 mL of PLGA (50 mg) dichloromethane solution, and then the sample was stirred conventionally for 150 s. Subsequently, the sample was mixed with 10 mL of polyvinyl alcohol aqueous solution (15 mg/mL), and conducted the conventional ultrasonic treatment for 50 s, followed by mixing with 300 mL of polyvinyl alcohol aqueous solution (8 mg/mL) and stirring conventionally until the complete volatilization of organic solvent (dichloromethane). And then, the sample was centrifuged at 10,000 rpm for 30 min and the supernatant was taken out. The precipitate was resuspended in 20 mL of sucrose aqueous solution (5 wt%), followed by freeze-drying at -80°C. And then, the sample was resuspended in 5 mL of normal saline and mixed with 3 mL of the above water-soluble component solution (10 mg/mL) and 0.5 mL of the non-water-soluble component solution (40 mg/mL), followed by standing for 20 min to obtain the activator nanoparticles loaded with the lysate components inside and on the surfaces.
   150 µL of the above water-soluble component solution (60 mg/mL) or 200 µL of the non-water-soluble component solution (10 mg/mL) was mixed with 100 µL of immunoadjuvant (CpG or poly I:C) solution (0.25 mg/mL), and then the mixture was added to 2 mL of PLGA (50 mg) dichloromethane solution, followed by stirring conventionally for 150 s. The sample was then mixed with 10 mL of polyvinyl alcohol aqueous solution (15 mg/mL) and conducted the conventional ultrasonic treatment for 50 s. Subsequently, the sample was mixed with 300 mL of polyvinyl alcohol aqueous solution (8 mg/mL), and stirred conventionally until the complete volatilization of organic solvent (dichloromethane). The sample was centrifuged at 10,000 rpm for 30 min and the supernatant was taken out. The sample was resuspended in 20 mL of sucrose aqueous solution (5 wt%), followed by freeze-drying at -80°C and resuspending in 5 mL of normal saline. And then, the sample was mixed with 2 mL of the above water-soluble component solution (10 mg/mL) and 0.5 mL of the non-water-soluble component solution (40 mg/mL), followed by standing for 20 min to obtain the activator nanoparticles loaded with lysate components inside and on the surfaces.
   The average particle size of micron particles before being loaded with the cell lysate components on the surfaces thereof was about 2.0 µm; the particle size of micron particles after being loaded with the cell lysate components on the surfaces thereof was about 2.1 µm; and 160 µg of cell lysate components were loaded on per mg of PLGA micron particles.
(3) Activation of tumor-specific T cells by micron particles: female C57BL/6 mice aged 6-8 weeks were selected to prepare melanoma-bearing mice. On day 0, 2,000,000 LLC lung tumor cells were inoculated subcutaneously into the lower right back of each mouse. On days 4, 7, 10, 15 and 20, the mice were subcutaneously injected with PBS or cancer nanovaccines loaded with whole-cell components of cancer cell. In the experiment, the tumor volumes of mice were recorded every three days from Day 6 and the tumor volume was calculated by the formula v = 0.52 × a × b², where v being the tumor volume, a being the tumor length, and b being the tumor width. On day 24, the mice in the PBS group and in the vaccine treatment group were sacrificed, followed by collecting the immune cells in their spleens.
   The cells were resuspended in an RPMI 1640 culture medium containing 10% FBS with a concentration of 5 × 10⁶ cells/mL. 100 µL of the above spleen cells were added to a 96-well plate that was pre-coated with an IFN-γ antibody a (capture antibody) and then the plate was sealed with a culture medium for more than 1 h. 25 µg of micron particles loaded with water-soluble components and 25 µg of micron particles loaded with non-water-soluble components were added to the cells, and then the sample was incubated at 37°C with 5% CO₂ for 72 h. And then, the mixture of cells and micron particles were discarded and the 96-well plate was washed, followed by adding an IFN-γ antibody b (detection antibody) and then incubating in an incubator at 37°C (5% CO₂) for more than 2 h. The solution containing the IFN-γ antibody b was discarded, the 96-well plate was washed, followed by using a corresponding method to develop color. Thus spots formed on the surface of 96-well plate and the ELISPOT analyzer was applied to read the data and analyze the experimental results.
   In the ELISPOT detection, the tumor-specific T cells were activated to secrete cell secretions such as IFN-γ, which would bind to the antibody a loaded on the 96-well plate; and after the addition of the antibody b, a double-antibody sandwich structure would be formed, and the detection antibody was connected with an enzyme that could assist in color development. When a substrate was added for color development, a spot would be formed at the location of each activated cell. The formation of a spot represented an activated tumor-specific T cell, so the number of tumor-specific T cells in the tested sample could be known by measuring the number of spots formed by color development in each well of the 96-well plate.
(4) Experimental results: The above experimental results of lung cancer were shown in Fig. 9. In fig. 9, a showed the inhibition effect of cancer vaccine treatment on the tumor growth rate (n ≥ 9), and b showed the content of activated tumor-specific T cells in the peripheral immune cells of peripheral spleen after the incubation with micron particles loaded with tumor tissue lysates analyzed by the ELISPOT detection.

As shown in Fig. 9, compared with the PBS blank control group, there were significantly more activated T cells after the peripheral immune cells of mice in the vaccine treatment group were co-incubated with the micron particles loaded with tumor whole-cell components or the micron particles together loaded with tumor whole-cell components and immunoadjuvants, which indicated that the content of tumor-specific T cells in the peripheral tissues of mice treated with the cancer vaccine increased significantly. Moreover, whether CpG or Poly(I:C) was used as an immunoadjuvant, after the co-incubation with peripheral immune cells, the micron particles loaded with tumor whole-cell components and immunoadjuvants could activate more T cells than the micron particles loaded with tumor whole-cell components. The above results indicated that adding immunoadjuvants could activate more cancer antigen-specific T cells.

The content of tumor-specific T cells in peripheral blood and other peripheral tissues of cancer patients is positively correlated with the prognosis of patients. The present invention collects whole-cell components of tumor cells or tumor tissues, and then co-incubates the free whole-cell components or the cell lysate components loaded on nano/micron particles with peripheral immune cells. After the tumor-specific T cells are activated, specific molecules of the tumor-specific T cells are detected, so that the content of the tumor-specific T cells in peripheral tissues, such as peripheral blood, can be determined. The inventiveness of the present invention lies in using cancer cells, tumor tissue whole cells, cancer cell lysate components or tumor tissue whole cell lysate components as activators to detect tumor-specific T cells in peripheral immune cells, with all the involved particle loading, cell incubation, specific secretion detection, etc. being present technologies in the field.

## Claims

1. A detection method for tumor-specific T cells, comprising the following steps: incubating activators with peripheral immune cells, and then detecting specific molecules of the tumor-specific T cells, thus achieving detection of the tumor-specific T cells, wherein the activators include tumor cells, tumor tissue whole cells, tumor cell lysate components, and tumor tissue whole-cell lysate components.

2. A detection method for the content of tumor-specific T cells, comprising the following steps: incubating activators with peripheral immune cells, then detecting specific molecules of the tumor-specific T cells, and then obtaining the content of tumor-specific T cells according to the ratio of the number of tumor-specific T cells to the number of peripheral immune cells, wherein the activators include tumor cells, tumor tissue whole cells, tumor cell lysate components, and tumor tissue whole-cell lysate components.

3. The detection method according to claim 1 or 2, **characterized in that** the lysate components are water-soluble and/or non-water-soluble lysate components.

4. The detection method according to claim 1 or 2, **characterized in that** the lysate components are in a free state, or are loaded on nano/micron particles.

5. The detection method according to claim 4, **characterized in that** the lysate components are loaded inside and/or on the surfaces of nano/micron particles.

6. The detection method according to claim 4, **characterized in that**: the nano/micron particles are made of organic materials, inorganic materials, or biological materials; and the nano/micron particles are nano particles or micron particles.

7. The detection method according to claim 1 or 2, **characterized in that** the incubation is carried out under the conditions that cells can survive.

8. The detection method according to claim 1 or 2, **characterized in that**: the tumors include blood tumors and solid tumors; and the specific molecules are proteins, peptides, nucleic acids, sugars, or lipids.

9. An application of the activators in detecting tumor-specific T cells in peripheral immune cells, wherein the activators include tumor cells, tumor tissue whole cells, tumor cell lysate components, and tumor tissue whole-cell lysate components.

10. The application according to claim 9, **characterized in that** the lysate components are in a free state, or are loaded on nano/micron particles.
